# EUROPEAN PATENT SPECIFICATION

(11) **EP 2 150 316 B1**
(45) Date of publication and mention of the grant of the patent: **27.06.2018**
(21) Application number: 08795825.2
(22) Date of filing: 30.04.2008
(51) Int. Cl.: A61Q 11/00, A61K 8/04, A61K 8/25, A61K 8/81

(54) **ORAL CARE COMPOSITION TO REDUCE OR ELIMINATE DENTAL SENSITIVITY**
ZUSAMMENSETZUNG GEGEN ZAHNEMPFINDLICHKEIT
COMPOSITION DE SOINS BUCCAUX DESTINÉE À RÉDUIRE OU À ÉLIMINER LA SENSIBILITÉ DENTAIRE

(30) Priority: 30.04.2007 US 742039; 16.04.2008 US 103919
(43) Date of publication of application: 10.02.2010
(73) Proprietor: Colgate-Palmolive Company, New York, NY 10022 (US)
(72) Inventor: ZAIDEL, Lynette, Cranford, NJ 07016 (US); CHOPRA, Suman, K., Monroe, NJ 08831 (US); PRENCIPE, Michael, Princeton Junction, NJ 08550 (US); WANG, Qin, Monmouth Junction, NJ 08852 (US); CHRISTOPOULOU, Constantina, Monroe, NJ 08831 (US)
(74) Representative: Wibbelmann, Jobst
(86) International application number: PCT/US2008/061925
(87) International publication number: WO 2008/140936

(56) References cited:
- WO-A-01/70178
- WO-A1-2005/065634
- US-A- 3 538 230
- US-A- 4 634 589
- US-A- 4 853 367
- US-A- 4 992 258
- US-A- 5 032 178
- US-A- 5 310 543
- US-A- 5 354 550
- US-A- 5 723 105
- US-A- 5 939 051
- US-A1- 2002 037 258
- US-A1- 2003 026 768
- US-A1- 2004 241 108
- US-A1- 2006 251 737
- US-B1- 6 447 756
- WERNER STÖBER, ARTHUR FINK, ERNST BOHN: "Controlled growth of monodisperse silica spheres in the micron size range" JOURNAL OF COLLOID AND INTERFACE SCIENCE, vol. 26, 1968, pages 62-69, XP002564574
- MILLER ET AL: "Evaluation of a new dentifrice for the treatment of sensitive teeth", JOURNAL OF CLINICAL DENTIS, PROFESSIONAL AUDIENCE COMMUNICATIONS, YARDLEY, PA, US, vol. 5, 1 January 1994 (1994-01-01), pages 71-79, XP009128272, ISSN: 0895-8831

## Description

### BACKGROUND OF THE INVENTION

Dentin is a portion of the tooth internal to the enamel and cementum that has a radially striated appearance owing to a large number of fine canals or tubules known as the dentinal tubules. Tubules run from the pulp cavity to the periphery of the dentin and are generally about two microns in diameter at their base and somewhat narrower at their periphery. Tubules are not usually exposed to the environment in the oral cavity, as they are usually covered by enamel or cementum. The cementum in turn is often covered by the gums.

It is commonly understood that partially or fully exposed tubules can lead to tooth sensitivity, an irritating and painful condition. In this theory, recession of the gum line exposes cementum to erosion. The eroded cementum in turn exposes the hollow dentinal tubules. The exposed tubules cause nerves within the tooth to be affected excessively by external oral stimuli because material and energy transfer between the exterior and interior of the tooth is accelerated through the tubules. Common environmental stimuli, such as heat, cold, chemicals and physical and mechanical pressure or stimuli, such as brushing, are able to irritate the nerve through the open dentin tubules and thereby create pain. The pain of sensitive teeth appears to result from these stimuli, which apparently cause fluid movements in the dentinal tubules that activate pulpal nerve endings.

Conventionally, two approaches have been taken to treat or ameliorate tooth sensitivity. Under one approach, the chemical environment proximal to the nerve is altered by application of various agents, such that the nerve is not stimulated, or not stimulated as greatly. Known agents useful in this chemical approach, including potassium salts (such as potassium nitrate, potassium bicarbonate, potassium chloride) and strontium, zinc salts, and chloride salts.

The second approach involves the mechanical shield of the nerve by, e.g., blocking of the dentinal tubules wholly or partially with "tubule blocking agents." Agents that have been disclosed in the prior art include, e.g., cationic alumina, clays, water-soluble or water-swellable polyelectrolytes, maleic acid copolymers and polyethylene particles. In particular both approaches have been combined by Miller, S. et al. as published in "Evaluation of a New Dentifrice for the Treatment of Sensitive Teeth" in The Journal of Clinical Dentistry, v. 5, p.71-79, 1994, Special Issue.

However, both the chemical and the mechanical approaches, because they require the incorporation of one or more additional materials to the dentifrice, may result in formulation difficulties, either technical or related to increased costs. For this reason there is a need in the art for a dentifrice that, upon use, prevents or reduces tooth sensitivity, yet is not associated with significant processing or formulation disadvantages.

### BRIEF SUMMARY OF THE INVENTION

The invention includes an oral care composition that reduces and/or eliminates the perception of tooth sensitivity.

The oral care composition as defined in claim 1 comprises:
a. an adherent material, which is a polymer having a number average molecular weight of 1,000 to 5,000;
b. silica particles having an average particle size of 3 to 4 microns, wherein the particles are present in the composition in an amount of 5% by weight or greater, and the composition provides a fluid flow rate of no greater than about 45% of the fluid flow rate of etched dentin. The compositions described provide a fluid flow rate of no greater than about 45% of the fluid flow value of etched dentin, representing at least a 55% reduction in dentin permeability.

Also included within the scope of the invention is the use of an oral care composition as defined in claim 12 for use in reducing dental sensitivity by occluding a dentin tubule within the surface of a mammalian tooth to which the composition has been applied.

### BRIEF DESCRIPTION OF THE DRAWINGS

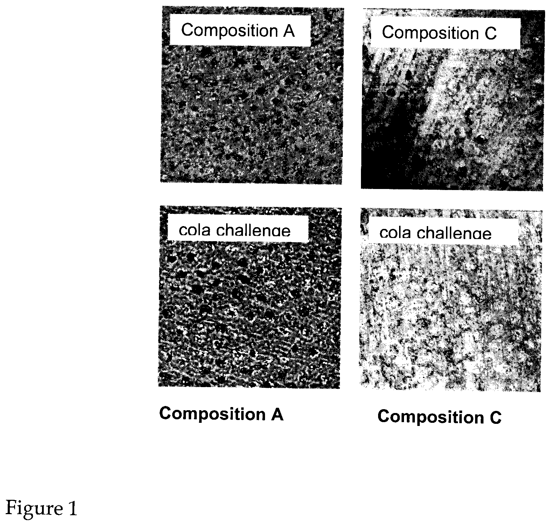

Figure 1 shows a comparison of the occlusion incidence of composition A versus composition C in an acid-treated mammalian tooth substrate.

### DETAILED DESCRIPTION OF THE INVENTION

The invention described herein includes an oral care composition as defined in claim 1 comprising:
a. an adherent material, which is a polymer having a number average molecular weight of 1,000 to 5,000;
b. silica particles having an average particle size of 3 to 4 microns, wherein the particles are present in the composition in an amount of 5% by weight or greater, and the composition provides a fluid flow rate of no greater than about 45% of the fluid flow rate of etched dentin. The compositions may contain additional therapeutic and non-therapeutic components, and may also be utilized in the practice of various methods, all of which are included within the scope of the invention. The composition within the scope of the invention may be useful in, for example, reducing or eliminating tooth sensitivity of a mammal, improving/maintaining systemic health, and/or occluding dentin tubules.

The oral compositions of the invention include an adherent material. The adherent material as defined in the claims may be any known or to be developed in the art that attaches
to the surface of a mammalian tooth and/or to the heterogenous biofilm which also may be present on a tooth's surface. Attachment may occur by any means, such as ionic interaction, van der Waals forces, hydrophobic-hydrophilic interactions, etc. The adherent material may be, for example, chitosan, chitin, a gum or a marine colloid. Other contemplated adherent materials include any homopolymers or copolymers (hereinafter referred to collectively as a "polymer") that adhere to the surface of a tooth. Such polymers may include silicone polymers, polymers having monomers of polyvinyl phosphonic acid, poly (1-phosphonopropene) sulfonic acid, poly(beta styrene phosphonic acid), alpha styrene phosphonic acid, synthetic anionic polymeric polycarboxylate, maleic anhydride, maleic acid, and methyl vinyl ether. Polymers having molecular weights 1,000 to 5,000 (number average) are used. In various embodiments, one may use a copolymer of methyl vinyl ether and maleic anhydride, in for example, a monomer ratio of about 1:4 to about 4:1. Other polymers that may be used as adherent materials include those recited in United States Patent Nos. 4,521,551; 4,485,090; 4,138,477; 4,138,914; and 3,956,480.

The oral compositions within the scope of the invention also include silica particles that have an average particle size of 3 to 4 microns, such that one or more particles is/are capable of becoming lodged within the tubule, thereby effecting a reduction or elimination of perceived tooth sensitivity.

The silica particle may be prepared by any means known or to be developed in the art, and may be surface modified, if desired, to increase the capacity of the particle to adhere to a tooth surface. Examples may be found in, *e.g.*, United States Patent Application Serial Number 11/271,306. The silica particle is present in the composition in an amount of about 5% or greater by weight of the total composition. Alternatively, the silica particle may be present in an amount of 5%, 10%, 15%, 20% or 25% by weight.

Any abrasive particulates may be used and may be selected from sodium bicarbonate, calcium phosphate (e.g.,dicalcium phosphate dihydrate), calcium sulfate, precipitated calcium carbonate, silica (e.g., hydrated silica), iron oxide, aluminium oxide, perlite, plastic particles, e.g., polyethylene, and combinations thereof. In particular, the abrasive may be selected from a calcium phosphate (e.g.,dicalcium phosphate dihydrate), calcium sulfate, precipitated calcium carbonate, silica (e.g., hydrated silica), calcium pyrophosphate and combinations. Any type of silica may be used, such as precipitated silicas or silica gels. Preferred are commercially available silicas such as INEOS AC43, available from Ineos Silicas, Warrington, United Kingdom.

The oral care compositions described herein may be formulated into any delivery form that permits contact of the adherent material and the particles, to the tooth surface. For example, the compositions may be formulated into a mouth rinse, a paste, a gel, a lozenge (dissolvable or chewable), a spray, a gum, and a film (wholly or partially dissolvable, or indissoluble). The composition may contain any conventional excipients or carriers, although these will vary depending on the dosage form or means of dosage selected. Excipents or carriers can include, for example, humectants, colorants, flavorants, glycerin, sorbitol, xylitol, and/or propylene glycol, water or other solvents, gum bases, thickening agents, surfactants, carrageenan (rich moss), xanthan gum and sodium carboxymethyl cellulose, starch, polyvinyl pyrrolidone, hydroxyethyl propyl cellulose, hydroxybutyl methyl cellulose, hydroxypropyl methyl cellulose, and hydroxyl ethyl cellulose and amorphous silicas.

Surfactants may be included, if desired. Examples of suitable surfactants include water-soluble salts of higher fatty acid monoglyceride monosulfates, such as the sodium salt of monosulfated monoglyceride of hydrogenated coconut oil fatty acids; higher alkyl sulfates such as sodium lauryl sulfate; alkyl aryl sulfonates such as sodium dodecyl benzene sulfonate; higher alkyl sulfoacetates, such as sodium lauryl sulfoacetate; higher fatty acid esters of 1, 2-dihydroxypropane sulfonate; and the substantially saturated higher aliphatic acyl amides of lower aliphatic amino carboxylic compounds, such as those having 12-16 carbons in the fatty acid, alkyl or acyl radicals; and the like. Examples of the last mentioned amides include N-lauryl sarcosine, and the sodium, potassium and ethanolamine salts of N-lauryl, N-myristoyl, or N-palmitoyl sarcosine. Others include, for example, nonanionic polyoxyethylene surfactants, such as Polyoxamer 407, Steareth 30, Polysorbate 20, and castor oil; and amphoteric surfactants, such as cocamidopropyl betaine (tegobaine), and cocamidopropyl betaine lauryl glucoside; condensation products of ethylene oxide with various hydrogen containing compounds that are reactive therewith and have long hydrocarbon chains (*e.g.*, aliphatic chains of from about 12 to about 20 carbon atoms), which condensation products (ethoxamers) contain hydrophilic polyoxyethylene moieties, such as condensation products of poly (ethylene oxide) with fatty acids, fatty alcohols, fatty amides and other fatty moieties, and with propylene oxide and polypropylene oxides.

In an embodiment, the oral composition includes a surfactant system that is sodium laurel sulfate (SLS) and tauranol. If desired, the SLS and tauranol may be present in a ratio of about 1:5 to about 1:3.

Dentin that is treated with the combination of the invention produce a fluid flow rate of no greater than about 45%, about 25%, about 20%, about 15% or about 10% of the flow rate value on the etched dentin, as determined by the Dentin Conductance Procedure.

Dentin Conductance Procedure: Extracted human molars are cut at the crown and roots using a diamond saw. The pulp is removed and the resulting dentin segment is stably mounted, such as onto an acrylic block. Tubing is connected from a hole in the acrylic block mounting just below the pulp chamber. The dentin segment is connected to an apparatus that measures the rate of fluid flow (hydraulic conductance).
See, Zhang et al., "The effects of pain free desensitizer on dentine permeability and tubule occlusion over time, in vitro", Journal of Clinical Periodontol, 25(11 Pt 1): 884-91 (Nov, 1998).

The top surface of the dentin is etched with citric acid. The fluid flow rate across the etched dentin is measured under 120 cm water pressure. The dentin surface is then brushed 2 minutes with a slurry of the oral composition of the invention diluted with 3 parts deionized water and the fluid flow rate is measured again. See Pashley et al., "Effects of desensitizing dentifrices in vitro," J. Periodontal., 55 (9): 522-525 (Sep, 1984).

The oral care composition may include any other therapeutic, cosmetic, and/or aesthetic materials as may be desired. Examples include desensitizing agents, a nitrate salt, an arginine ester, a bicarbonate salt, potassium nitrate, and an arginine-bicarbonate-phytate complex, a chemical whitening agent (such as a peroxide releasing compound), an opaque whitening agent (such as hydroxyapetite) and an anticalculus agent. Other options for inclusion in the oral care composition of the invention include triclosan; stannous ion agents; chlorhexidine; alexidine; hexetidine; sanguinarine; benzalkonium chloride; salicylanilide; domiphen bromide; cetylpyridinium chloride (CPC); tetradecylpyridinium chloride (TPC); N-tetradecyl-4-ethylpyridinium chloride (TDEPC); octenidine; delmopinol; octapinol; nisin; zinc ion agents; copper ion agents; essential oils; furanones; bacteriocins, ethyl lauroyl arginate, extracts of magnolia, a metal ion source, arginine bicarbonate, honokiol, magonol, ursolic acid, ursic acid, morin, extract of sea buckthorn, an enzyme, a *Camellia* extract, a flavonoid, a flavan, halogenated diphenyl ether, creatine, and propolis.

The oral care composition of the invention may be prepared by any means known in the art. For example, preparation methods for dentifrices are well known, for example, as described in United States Patent Nos. 3,966,863; 3,980,767; 4,328,205; and 4,358,437. In general, any humectant (e.g., glycerin, sorbitol, propylene glycol, and/or polyethylene glycol) is dispersed in water in a conventional mixer under agitation. Into that dispersion are added the thickeners, such as carboxyl methyl cellulose (CMC), carrageenan, or xanthan gum; any anionic polycarboxylate; any salts, such as sodium fluoride anticaries agents; and any sweeteners.

The resultant mixture is agitated until a homogeneous gel phase is formed. Into the gel phase are added any pigments utilized, such as TiO₂, and additionally any acid or base required to adjust the pH of the composition. These ingredients are mixed until a homogeneous phase is obtained.

The mixture is then transferred to a high speed/vacuum mixer, wherein the surfactant ingredients are added to the mixture. The silicas utilized are added subsequently. Any water insoluble agents, such as triclosan, are solubilized in the flavor oils to be included in the dentifrice, and that solution is added along with the surfactants to the mixture, which is then mixed at high speed for about 5 to about 30 minutes, under a vacuum of about 20 to about 50 mm of Hg. The resultant product is a homogeneous, semi-solid, extrudable paste or gel product.

Tests were utilized to analyze the structure of initially produced silica gel during the overall in situ gel/precipitate production method. Included within these analyses was porosity. Such a property of accessible porosity was obtained using nitrogen adsorption-desorption isotherm measurements. The BJH (Barrett-Joiner-Halender) model average pore diameter was determined based on the desorption branch utilizing an Accelerated Surface Area and Porosimetry System (ASAP 2010) available form Micromeritics Instrument Corporation, Norcross, Ga. Samples were out-gassed at 150-200.degree. C. until the vacuum pressure was about 5 mu.m of Mercury. Such an analyzer was an automatic volumetric type at 77 K. Pore volume was obtained at a pressure P/P.sub.0=0.99. Average pore diameter is derived from pore volume and surface area assuming cylindrical pores. Pore size distribution (.DELTA.V/.DELTA.D) was calculated using the BJH method, which provides the pore volume within a range of pore diameters. A Halsey thickness curve type was used with pore size range of 1.7 to 300.0 nm diameter, with zero fraction of pores open at both ends. The particles of the invention also have porosity such that when an aqueous dispersion of the particles is dried less than 0.45 cc/g of pore volume as measured by BJH nitrogen porosimetry is from pores having a pore size of 600 Angstroms or smaller.
The invention also includes an oral care composition comprising: a. an adherent material; b. silica particles having an average particle size of 3 to 4 microns, wherein the particles are present in the composition in an amount of 5% by weight or greater, and the composition provides a fluid flow rate of no greater than about 45% of the fluid flow rate of etched dentin, preferably, an oral care composition according to claim 1, for use in reducing dental sensitivity by occluding a dentin tubule within the surface of a mammalian tooth to which the composition has been applied.

The method includes the steps of applying any of the compositions described above to the tooth surface. Application may be carried out by any method, so long as the adherent material and the particles are placed in contact with the tooth surface. Application may be accomplished by brushing, flossing, irrigating, wiping, rinsing (lavage of oral cavity), foam/gel and in-tray application, masticating, spraying, painting, etc., or applied by film or strip.

The compositions can also be used to increase or maintain the systemic health of a mammal by applying a composite to an oral surface (both hard and soft tissues of the oral cavity). The composition for use in this method may be any described above, provided that it contains at least one of triclosan; triclosan monophosphate; chlorhexidine; alexidine; hexetidine; sanguinarine; benzalkonium chloride; salicylanilide; domiphen bromide; cetylpyridinium chloride (CPC); tetradecylpyridinium chloride (TPC); N-tetradecyl-4-ethylpyridinium chloride (TDEPC); octenidine; delmopinol; octapinol; nisin; zinc ion agent; copper ion agent; essential oils; furanones; bacteriocins, ethyl lauroyl arginate, extracts of magnolia, a metal ion source, arginine bicarbonate, honokiol, magonol, ursolic acid, ursic acid, morin, extract of sea buckthorn, a peroxide, an enzyme, a *Camellia* extract, a flavonoid, a flavan, halogenated diphenyl ether, creatine, and propolis. The application may be at least once a day, although up to five times per day may be preferred, and may be carried out over a duration of time, *e.g.,* one week, up to one year, up to three years or for a lifetime.

### Example 1

Two compositions paste-form was prepared using the materials and amounts set out in Table I and the process described below. Compositions B-D represents a composition within the scope of the invention and composition A is a control composition that does not contain the specified silica particle. Ineos AC43 silica has a pore volume of 0.29 cm³ (cc)/g.

**Table I**

| **Ingredient** | **A** | **B** | **C** | **D** |
|---|---|---|---|---|
| Water | OS | OS | OS | OS |
| Saccharin | 0.3 | 0.3 | 0.3 | 0.3 |
| NaF | 0.243 | 0.243 | 0.243 | 0.243 |
| Glycerin | 20 | 20 | 20 | 20 |
| Propylene Glycol | 0.5 | 0.5 | 0.5 | 0.5 |
| Carboxy methyl cellulose (CMC) | 1.1 | 1.1 | 1.1 | 1.1 |
| Iota Carrageenan | 0.4 | 0.4 | 0.4 | 0.4 |
| TiO2 | 0.5 | 0.5 | 0.5 | 0.5 |
| Sorbitol | 20.85 | 20.85 | 2 0.85 | 20.85 |
| PMV/MA Copolymer 13% soln | 15 | 15 | 15 | 15 |
| NaOH | 1.2 | 1.2 | 1.2 | 1.2 |
| Thickening silicas | 1.5 | 1.5 | 1.5 | 1.5 |
| Abrasive silicas | 20 | 17 | 15 | 11 |
| Ineos AC43 small particle silica | 0 | 3 | 5 | 9 |
| Flavor | 1 | 1 | 1 | 1 |
| Triclosan | 0.3 | 0.3 | 0.3 | 0.3 |
| Sodium laureth sulfate | 1.5 | 1.5 | 1.5 | 1.5 |
| Total | 100 | 100 | 100 | 100 |

Sodium saccharin and sodium fluoride was dissolved in water. Triclosan was dissolved in flavor.

Glycerin and propylene glycol were mixed together. Sodium CMC and iota carragenan was dispersed. Titanium dioxide was added to the mixture. This was followed by the addition of sorbitol. To this sodium saccharin and sodium fluoride in water was added and it was mixed for 15 minutes at 49°C. Then the PMV/MA copolymer and sodium hydroxide (50%) were added at 49°C (5 minutes mixing). The whole mixture was dropped into a mixer and mixed. Subsequently the abrasive silicas and the Ineos AC43 silica particles were added at high speed under full vacuum.

Premix flavor and triclosan and sodium sulphate powder were added. It was mixed for 10 minutes at medium speed under full vacuum. The vacuum was released and the whole batch was inspected for uniformity.

Fluid flow across dentin samples using each composition (A-D) was measured using the procedure described above.

| Composition | %Flow vs. etched baseline |
|---|---|
| A (0% AC43 silica)* | 92 ± 2 |
| B (3% AC43 silica)* | 77 ± 8 |
| C (5% AC43 silica) | 22 ± 4 |
| D (9% AC43 silica) | 5 ± 1 |

| | |
|---|---|
| * not according to the invention | |

Dentin treated with compositions C-D (polymer and small particle silica) produced a fluid flow rate that was 5-22% of the fluid flow value of etched dentin which was significantly lower than that of composition A with polymer alone. Values for typical commercial dentifrices without the small particle silica/polymer would be 50-100% of the value of etched dentin (ref: Pashley DH et al, Effect of desensitizing dentrifices. J. Periodontol, 1984: 55: 522-525). Thus, compositions C-D produced significant reductions in fluid flow rate.

Similarly, confocal microscopy images taken of etched dentin treated with Composition C showed significant occlusion/coating of the open dentin tubules when compared to etched dentin treated with Composition A. In addition, the occlusive coating produced by Composition C was resistant to acid dissolution by cola.

## Claims

1. An oral care composition comprising:
a. an adherent material, which is a polymer having a number average molecular weight of 1,000 to 5,000;
b. silica particles having an average particle size of 3 to 4 microns, wherein the particles are present in the composition in an amount of 5% by weight or greater, and the composition provides a fluid flow rate of no greater than about 45% of the fluid flow rate of etched dentin.

2. The composition of claim 1, wherein the particles have a porosity of less than 0.45 cm³ (cc)/g in pores of 60 nm (600 Angstroms) or smaller.

3. The composition of claim 1, wherein the adherent material is selected from polymers of polyvinyl phosphonic acid, poly (1-phosphonopropene) sulfonic acid, poly(beta styrene phosphonic acid), alpha styrene phosphonic acid, synthetic anionic polymeric polycarboxylate, maleic anhydride, maleic acid, and methyl vinyl ether.

4. The composition of claim 1, wherein the adherent molecule is a polymer of methyl vinyl ether and maleic anhydride.

5. The composition of claim 1, wherein the composition is formulated into a form selected from a rinse, a paste, a gel, a gum, a dissolvable lozenge, and a film, optionally which film is a dissolvable film.

6. The composition of claim 1 further comprising a desensitizing agent, optionally selected from a nitrate salt, an arginine ester, a bicarbonate salt, potassium nitrate, and an arginine-bicarbonate-phytate complex.

7. The composition of claim 1 further comprising an antibacterial agent.

8. The composition of claim 1 further comprising an agent selected from a chemical whitening agent, an opaque whitening agent and an anticalculus agent.

9. The composition of claim 1 further comprising triclosan.

10. The composition of claim 1 further comprising an agent selected from a stannous ion agent; chlorhexidine; alexidine; hexetidine; sanguinarine; benzalkonium chloride; salicylanilide; domiphen bromide; cetylpyridinium chloride (CPC); tetradecylpyridinium chloride (TPC); N-tetradecyl-4-ethylpyridinium chloride (TDEPC); octenidine; delmopinol; octapinol; nisin; zinc ion agent; copper ion agent; essential oils; furanones; bacteriocins, ethyl lauroyl arginate, extracts of magnolia, a metal ion source, arginine bicarbonate, honokiol, magonol, ursolic acid, ursic acid, morin, extract of sea buckthorn, a peroxide, an enzyme, a *Camellia* extract, a flavonoid, a flavan, halogenated diphenyl ether, creatine, and propolis.

11. The composition of claim 1, wherein the particles are present in the composition in an amount of from 5 to 25% by weight.

12. An oral care composition comprising:
a. an adherent material;
b. silica particles having an average particle size of 3 to 4 microns, wherein the particles are present in the composition in an amount of 5% by weight or greater, and the composition provides a fluid flow rate of no greater than about 45% of the fluid flow rate of etched dentin, preferably, an oral care composition according to claim 1, for use in reducing dental sensitivity by occluding a dentin tubule within the surface of a mammalian tooth to which the composition has been applied.

## Patentansprüche

1. Mundpflegezusammensetzung, umfassend:
a. ein Haftmaterial, das ein Polymer mit einem Zahlenmittel des Molekulargewichts von 1000 bis 5000 ist;
b. Silicapartikel mit einer mittleren Partikelgröße von 3 bis 4 Mikrometern, wobei die Partikel in der Zusammensetzung in einer Menge von 5 Gew.-% oder größer vorliegen, und die Zusammensetzung eine Fluidflussrate von nicht größer als etwa 45% der Fluidflussrate von geätztem Dentin bereitstellt.

2. Zusammensetzung nach Anspruch 1, wobei die Partikel eine Porosität von weniger als 0,45 cm³ (cc)/g in Poren von 60 nm (600 Angström) oder kleiner aufweisen.

3. Zusammensetzung nach Anspruch 1, wobei das Haftmaterial aus Polymeren von Polyvinylphosphonsäure, Poly(1-phosphonopropen)sulfonsäure, Poly(beta-styrolphosphonsäure), alpha-Styrolphosphonsäure, synthetischem anionischen polymeren Polycarboxylat, Maleinsäureanhydrid, Maleinsäure und Methlvinylether ausgewählt ist.

4. Zusammensetzung nach Anspruch 1, wobei das haftende Molekül ein Polymer aus Methylvinylether und Maleinsäureanhydrid ist.

5. Zusammensetzung nach Anspruch 1, wobei die Zusammensetzung in einer Form formuliert ist, die aus einer Spülung, einer Paste, einem Gel, einem Gummi, einer löslichen Lutschtablette und einem Film ausgewählt ist, gegebenenfalls wobei der Film ein löslicher Film ist.

6. Zusammensetzung nach Anspruch 1, die weiterhin ein Desensibilisierungsmittel umfasst, gegebenenfalls ausgewählt aus einem Nitratsalz, einem Argininester, einem Bicarbonatsalz, Kaliumnitrat und einem Arginin-Bicarbonat-Phytat-Komplex.

7. Zusammensetzung nach Anspruch 1, die weiterhin ein antibakterielles Mittel umfasst.

8. Zusammensetzung nach Anspruch 1, die weiterhin ein Mittel umfasst, das aus einem chemischen Aufhellungsmittel, einem deckenden Aufhellungsmittel und einem Anti-Zahnsteinmittel ausgewählt ist.

9. Zusammensetzung nach Anspruch 1, die weiterhin Triclosan umfasst.

10. Zusammensetzung nach Anspruch 1, die weiterhin ein Mittel umfasst, das aus einem Zinnionenmittel; Chlorhexidin; Alexidin; Hexetidin; Sanguinarin; Benzalkoniumchlorid; Salicylanilid; Domiphenbromid; Cetylpyridiniumchlorid (CPC); Tetradecylpyridiniumchlorid (TPC); N-Tetradecyl-4-ethylpyridiniumchlorid (TDEPC); Octenidin; Delmopinol; Octapinol; Nisin; Zinkionenmittel; Kupferionenmittel; ätherischen Ölen; Furanonen; Bacteriocinen, Ethyllauroylarginat, Magnolienextrakten, einer Metallionenquelle, Argininbicarbonat, Honokiol, Magonol, Ursolsäure, Ursinsäure, Morin, Sanddornextrakt, einem Peroxid, einem Enzym, einem Kamelienextrakt, einem Flavonoid, einem Flavan, halogeniertem Diphenylether, Kreatin und Propolis ausgewählt ist.

11. Zusammensetzung nach Anspruch 1, wobei die Partikel in der Zusammensetzung in einer Menge von 5 bis 25 Gew.-% vorliegen.

12. Mundpflegezusammensetzung, umfassend:
a. ein Haftmaterial;
b. Silicapartikel mit einer mittleren Partikelgröße von 3 bis 4 Mikrometern, wobei die Partikel in der Zusammensetzung in einer Menge von 5 Gew.-% oder größer vorliegen, und die Zusammensetzung eine Fluidflussrate von nicht größer als etwa 45% der Fluidflussrate von geätztem Dentin bereitstellt, bevorzugt eine Mundpflegezusammensetzung gemäß Anspruch 1, zur Verwendung beim Verringern von Zahnempfindlichkeit durch Verschließen eines Dentintubulus innerhalb der Oberfläche eines Säugerzahns, auf den die Zusammensetzung angewendet wurde.

## Revendications

1. Composition de soin buccal comprenant :
a. un matériau adhérent, qui est un polymère ayant un poids moléculaire moyen en nombre de 1 000 à 5 000 ;
b. des particules de silice ayant une taille moyenne de particule de 3 à 4 microns, dans laquelle les particules sont présentes dans la composition en une quantité de 5 % en poids ou plus, et la composition fournit un débit de fluide non supérieur à environ 45 % du débit de fluide de la dentine qui a été attaquée chimiquement.

2. Composition selon la revendication 1, dans laquelle les particules ont une porosité inférieure à 0,45 cm³ (cc)/g dans des pores de 60 nm (600 Angströms) ou moins.

3. Composition selon la revendication 1, dans laquelle le matériau adhérent est choisi parmi les polymères d'acide polyvinyl phosphonique, d'acide poly(1-phosphonopropène)sulfonique, de poly(acide bêta styrène phosphonique), d'acide alpha styrène phosphonique, de polycarboxylate polymère anionique synthétique, d'anhydride maléique, d'acide maléique et d'éther méthylvinylique.

4. Composition selon la revendication 1, dans laquelle la molécule adhérente est un polymère d'éther méthylvinylique et d'anhydride maléique.

5. Composition selon la revendication 1, dans laquelle la composition est formulée sous une forme choisie parmi un produit de rinçage, une pâte, un gel, une gomme, une pastille soluble et un film, éventuellement un film qui peut être dissout.

6. Composition selon la revendication 1, comprenant en outre un agent désensibilisant, éventuellement choisi parmi un sel de nitrate, un ester d'arginine, un sel de bicarbonate, un nitrate de potassium et un complexe d'arginine-bicarbonate-phytate.

7. Composition selon la revendication 1, comprenant en outre un agent antibac-térien.

8. Composition selon la revendication 1, comprenant en outre un agent choisi parmi un agent de blanchiment chimique, un agent de blanchiment opaque et un agent antitartre.

9. Composition selon la revendication 1, comprenant en outre du triclosan.

10. Composition selon la revendication 1 comprenant en outre un agent choisi parmi un agent d'ion stanneux ; la chlorhexidine ; l'alexidine ; l'hexétidine ; la sanguinarine ; le chlorure de benzalkonium ; le salicylanilide ; le bromure de domiphène ; le chlorure de cétylpyridinium (CPC) ; le chlorure de tétradécylpyridinium (TPC) ; le chlorure de N-tétradécyl-4-éthylpyridinium (TDEPC) ; l'octénidine ; le delmopinol ; l'octapinol ; la nisine ; un agent d'ion zinc ; un agent d'ion cuivre ; les huiles essentielles ; les furanones ; les bactériocines, l'éthyl lauroyl arginate, les extraits de magnolia, une source d'ions métalliques, le bicarbonate d'arginine, l'honokiol, le magonol, l'acide ursolique, l'acide ursique, la morine, un extrait d'argousier, un peroxyde, une enzyme, un extrait de camélia, un flavonoïde, un flavan, un éther diphénylique halogéné, la créatine et la propolis.

11. Composition selon la revendication 1, dans laquelle les particules sont présentes dans la composition en une quantité de 5 à 25 % en poids.

12. Composition de soin buccal comprenant :
a. un matériau adhérent ;
b. des particules de silice ayant une taille moyenne de particule de 3 à 4 microns, dans laquelle les particules sont présentes dans la composition en une quantité de 5 % en poids ou plus, et la composition fournit un débit de fluide non supérieur à environ 45 % du débit de fluide de la dentine qui a été attaquée chimiquement, de préférence, une composition de soin buccal selon la revendication 1, pour une utilisation dans la réduction de la sensibilité dentaire par occlusion d'un tubule dentinaire dans la surface d'une dent de mammifère sur laquelle la composition a été appliquée.
